# EUROPÄISCHE PATENTSCHRIFT

(11) **EP 2 077 870 B1**
(45) Veröffentlichungstag und Bekanntmachung des Hinweises auf die Patenterteilung: **30.04.2014**
(21) Anmeldenummer: 07802220.9
(22) Anmeldetag: 07.09.2007
(51) Int. Cl.: A61M 1/36

(54) **VERFAHREN ZUR UNTERSTÜTZUNG VON IMMUNTHERAPIEN**
METHOD FOR PROMOTING IMMUNOTHERAPIES
PROCÉDÉ POUR RENFORCER DES IMMUNOTHÉRAPIES

(30) Priorität: 07.09.2006 DE 102006042012
(43) Veröffentlichungstag der Anmeldung: 15.07.2009
(60) Teilanmeldung: 13163421.4
(73) Patentinhaber: Fresenius Medical Care Deutschland GmbH, 61352 Bad Homburg (DE)
(72) Erfinder: HEPPER, Martin, 67435 Neustadt (DE); LEINENBACH, Hans, Peter, 66636 Tholey (DE); NOCKEN, Frank, 60320 Frankfurt (DE)
(74) Vertreter: Stolmár, Matthias
(86) Internationale Anmeldenummer: PCT/EP2007/007840
(87) Internationale Veröffentlichungsnummer: WO 2008/028680

(56) Entgegenhaltungen:
- EP-A- 0 082 345
- US-B1- 6 406 861
- BORBERG ET AL: "Quo vadis haemapheresis" TRANSFUSION AND APHERESIS SCIENCE, ELSEVIER SCIENCE, LONDON, GB, Bd. 34, Nr. 1, Februar 2006 (2006-02), Seiten 51-73, XP005288266 ISSN: 1473-0502
- MONTGOMERY R A ET AL: "Plasmapheresis and intravenous immune globulin provides effective rescue therapy for refractory humoral rejection and allows kidneys to be successfully transplanted into cross-match-positive recipients." TRANSPLANTATION 27 SEP 2000, Bd. 70, Nr. 6, 27. September 2000 (2000-09-27), Seiten 887-895, XP002457983 ISSN: 0041-1337
- KIEWE P ET AL: "PHASE I TRIAL OF THE TRIFUNCTIONAL ANTI-HER2 X ANTI-CD3 ANTIBODY ERTUMAXOMAB IN METASTATIC BREAST CANCER" CLINICAL CANCER RES, Bd. 12, Nr. 10, 15. Mai 2006 (2006-05-15), Seiten 3085-3091, XP008067039 ISSN: 0732-183X
- TAYLOR RONALD P ET AL: "Drug insight: the mechanism of action of rituximab in autoimmune disease--the immune complex decoy hypothesis." NATURE CLINICAL PRACTICE. RHEUMATOLOGY FEB 2007, Bd. 3, Nr. 2, Februar 2007 (2007-02), Seiten 86-95, XP009091912 ISSN: 1745-8382
- BROWNING JEFFREY L: "B cells move to centre stage: novel opportunities for autoimmune disease treatment.", NATURE REVIEWS. DRUG DISCOVERY JUL 2006, vol. 5, no. 7, July 2006 (2006-07), pages 564-576, ISSN: 1474-1776
- TYDÉN GUNNAR ET AL: "ABO incompatible kidney transplantations without splenectomy, using antigen-specific immunoadsorption and rituximab.", AMERICAN JOURNAL OF TRANSPLANTATION : OFFICIAL JOURNAL OF THE AMERICAN SOCIETY OF TRANSPLANTATION AND THE AMERICAN SOCIETY OF TRANSPLANT SURGEONS JAN 2005, vol. 5, no. 1, January 2005 (2005-01), pages 145-148, ISSN: 1600-6135
- RECH J ET AL: "Combination of immunoadsorption and CD20 antibody therapy in a patient with mixed connective tissue disease.", RHEUMATOLOGY (OXFORD, ENGLAND) APR 2006, vol. 45, no. 4, April 2006 (2006-04), pages 490-491, XP009173279, ISSN: 1462-0324
- RECH J ET AL: "Immunoadsorption and CD20 antibody treatment in a patient with treatment resistant systemic lupus erythematosus and preterminal renal insufficiency.", April 2006 (2006-04), ANNALS OF THE RHEUMATIC DISEASES APR 2006, VOL. 65, NR. 4, PAGE(S) 552 - 553 ISSN: 0003-4967

## Beschreibung

Die vorliegende Erfindung betrifft ein Verfahren zur Steigerung der Effizienz von Immuntherapien und/oder zur Steigerung der Effizienz von Kombinationstherapien von Arzneimitteltherapien mit Immuntherapien. Immuntherapien werden insbesondere in der Krebsbehandlung eingesetzt.

Neben der konventionellen Krebsbehandlung, die auf chirurgischen Maßnahmen, der Verwendung chemotherapeutischer Medikamente und/oder Bestrahlung basiert, hat sich die Immuntherapie mit Antikörpern etabliert. Gleichermaßen sind eine Vielzahl biotechnologischer Produkte, zu denen unter anderen auch die therapeutischen Antikörper zählen, zur Behandlung von Autoimmunerkrankungen als Therapieoption bereits anerkannt. Sogenannte HIV-neutralisierende Antikörper werden derzeit zur Behandlung von AIDS in klinischen Studien untersucht.

Die Zielstrukturen, die sogenannten Antigene, sind für die verschiedenen Erkrankungen sehr unterschiedlich und auch die Wirkweise der jeweiligen Antikörpertherapien kann in Abhängigkeit von diesen Zielstrukturen sehr verschieden sein. Oberflächenantigene, die als Zielstruktur zur Behandlung von malignen Erkrankungen dienen, sind u.a. überexprimierte, Krebs-assoziierte, membranständige Proteine (EGFR/HER2/VEGFR) oder auch Zell-spezifische Proteine (CD20/CD52). Die Anzahl der Antigen-positiven Zellen ist bei der Entwicklung von Lymphomen überaus groß, so dass eine Verminderung dieser Zellpopulation ein anerkanntes therapeutisches Ziel darstellt. Neben der Behandlung von Lymphomen eignet sich die zielgerichtete Depletion Antigenpositiver Zellen auch zur Behandlung von Autoimmunerkrankungen und gegebenenfalls zur Unterdrückung der Immunantwort wie z.B. bei der Transplantation. Antikörper oder auch Fusionsproteine, gerichtet gegen zelluläre Botenstoffe wie den löslichen Tumornekrosefaktor, sind wichtige Medikamentezur Behandlung von Autoimmunerkrankungen.

Die ersten modernen monoklonalen Antikörper (Immunglobuline meistens des IgG1-typus) waren murinen Ursprungs, d.h. sie wurden mittels Mäuse- oder Rattenhybridomzellinien hergestellt. Diese IgG1 Moleküle werden jedoch vom Körper des Menschen als fremd erkannt, so dass sie durch das menschliche Immunsystem neutralisiert werden. Aus diesem Grund wurden modernere, sogenannte chimäre Antikörper, bestehend aus murinen Anteilen und menschlichen Anteilen in der IgG Struktur, hergestellt. Die sogenannte Humanisierung, bis hin zur biotechnologisch optimierten Variante der kompletthumanen Antikörper, stellt den nächsten Schritt zur weiteren Minimierung der murinen Anteile dar. Chimäre, humanisierte und humane IgG1-Antikörper können über einen längeren Zeitraum, beispielsweise über Monate, während der Therapie eingesetzt werden. (Abdullah N., Cancer Immunther. 48, 517-524), (Adams GP, Weiner LM, Nature Biotechnology, 2005; 23(9): 1146 - 1157).

Außerdem werden Immuntherapien unter anderem mit Fcy-Rezeptor-bindenden Agenzien durchgeführt. Fcγ-Rezeptoren (FcR) sind eine Familie von Rezeptoren, die spezifisch für die Fc-Teile von Immunoglobulin (IgG) sind. Diese Rezeptoren haben wichtige Aufgaben im normalen Immunsystem und dessen Widerstandsfähigkeit bei Infektionen. Somit sind IgG eine Klasse von Molekülen, die den Fcγ-Rezeptor binden.

Rezeptoren gibt es für jede Immunoglobulin-Klasse. Sie sind definiert durch die Klasse von Immunglobulin, an die sie binden. Beispielsweise bindet Fcγ-Rezeptor (FcγR) IgG, der Fcε-Rezeptor (FcεR) bindet IgE usw. Unter den FcyR-Rezeptoren werden drei Mitglieder von Unterfamilien unterschieden: FcγRI, der ein Rezeptor mit hoher Affinität für IgG ist, FcγRII, die Rezeptoren mit geringer Affinität für IgG sind, die jedoch gut an Aggregate von Immunkomplexen binden, und FcγRIII die Rezeptoren mit niedriger Affinität sind, die an Immunkomplexe binden.

Zwar sind alle diese Rezeptoren strukturell untereinander verwandt, sie haben jedoch verschiedene Aufgaben.

FcγR werden von den meisten hämatopoietischen Zellen exprimiert und spielen über die Bindung an IgG eine Schlüsselrolle bei der Homeostase des Immunsystems und dem Schutz gegen Infektionen. Insbesondere FcγRII ist ein Rezeptor mit niedriger Affinität für IgG, der im wesentlichen nur an IgG-Immunkomplexe bindet, und er wird auf einer Vielzahl von Zellstücken exprimiert, umfassend beispielsweise Monozyten, Makrophagen, Neutrophile, Eosinophile, Plättchen und B-Lymphozyten.

Fcγ-Rezeptoren sind in verschiedene Immun- und Entzündungsantworten einschließlich der Antikörper-abhängigen, zellvermittelten Zytotoxizität (ADCC), involviert. Die Antikör-per-vermittelte, zelluläre Zytotoxizität (ADCC) ist für die Wirkung biologischer Arzneimittel wie z.B. von poly- und monoklonalen Antikörpern, aber auch von Fusionsproteinen, verantwortlich. Die Effektivität der ADCC steht in direktem Zusammenhang mit der beschriebenen Interaktion der konstanten Region des Antikörpers, oder aber auch mit den Bindungseigenschaften des entsprechenden Proteins mit den Fcy-Rezeptoren. Für die ADCC scheint hierbei vor allem die Bindung an die aktivierenden Fcγ I und III Rezeptoren zu sein; wohingegen die Bindung eines Arzneimittels vorwiegend an FcγII Rezeptoren die Immunantwort unterdrückt.

Ein kontinuierlicher Einsatz therapeutischer Proteine, wie z.B. von Antikörpern, bzw. von FcR bindenden Agenzien, insbesondere zur Behandlung von Krebs- und Autoimmunerkrankungen, Infektionskrankheiten und zur Unterdrückung von Transplantationsabstossungsreaktionen, wird dadurch eingeschränkt, dass diese Medikamente in hohen Dosen während einer kontinuierlichen Therapie appliziert werden müssen.

Bei der Anwendung von Antikörpern einer anderen, aber sogar auch humanisierter oder humaner Antikörper, werden diese Fremdproteine vom Immunsystem des Patienten erkannt und in Abhängigkeit von der jeweiligen Immunogenität durch die Entwicklung körpereigener (autologer) Antikörper (Humane anti-Spezies Antikörper) neutralisiert. Diese Immunantwort wird durch z.B. Humane Anti-Murine Antikörper (HAMA) im Falle muriner Antikörper, HARA im Falle von Antikörpern aus Kaninchen, (HAMA) im Falle muriner Antikörper bzw. durch Humane Anti-Humane Antikörper (HAHA) im Falle humanisierter oder humaner Antikörper vermittelt. Selbstverständlich induzieren auch mono- und polyklonale Antikörperprodukte, die aus anderen Spezies wie z.B. Ratten, Pferden, Ziegen, Schafen, Rinder oder auch Schweinen gewonnen werden, die Entwicklung von humanen Antikörpern gerichtet gegen die jeweilige Spezies, was oft zu starken Immunreaktionen und zu einem Verlust an Effizienz führen kann. Diese Form der körpereigenen Antikörper gegen Antikörper aus anderen Organismen werden zusammenfassend humane Anti-Spezies-Antikörper genannt.

Die Immunogenität der körperfremden, biotechnologischen Produkte beschränkt deren therapeutische Effektivität z.B. durch die Entwicklung der oben beschriebenen HAMA und HAHA Antwort des Immunsystems.

Die *in vivo* beobachtete relativ niedrige Wirksamkeit therapeutischer Antikörperpräparate steht in starkem Widerspruch zur oftmals sehr hohen Antikörper-vermittelten, zellulären Zytotoxizität (ADCC), die in vorherigen in vitro Untersuchungen nachgewiesen worden wurde. Dieses Phänomen tritt auch bei Kombinationstherapien herkömmlicher Arzneimitteltherapien, z.B. Chemotherapien, mit Immuntherapien auf. Wenn im folgenden von Arzneimitteln allgemein gesprochen wird, so sind damit herkömmliche, nicht immuntherapeutisch wirkende Arzneimittel gemeint, ganz besonders werden vorliegend darunter Chemotherapeutika und/oder Cytostatika verstanden. Eine mögliche Erklärung für dieses Phänomen stellt die beschriebene Inhibierung der therapeutischen Antikörper durch HAMA/HAHA dar (Preithner, S. et al. in Molecular Immunology, 43 (2006) 1183-1193.) Des weiteren stellen die natürlich vorkommenden IgG1 im Patientenblut Moleküle dar, die vergleichbar mit den therapeutischen Antikörpern um die entsprechenden Fc-Rezeptoren in Wettbewerb stehen, da auch diese IgG1 an die entsprechenden Rezeptoren binden und diese dadurch besetzen.

Es wurde erwartet, dass humanisierte oder humane Antikörper zu einer verbesserten Wirksamkeit und zu einem besseren Sicherheitsprofil führen würden. Wie vorstehend schon erwähnt, zeigen überraschenderweise auch fast alle humanisierten oder humanen Antikörper eine dramatisch verminderte Wirksamkeit in vivo, verglichen mit ihrer in vitro-Aktivität.

Daher ist es nötig, vielfach höhere Dosen von Antikörpern den Patienten zu verabreichen wodurch das Risiko unerwünschter Nebenwirkungen erhöht wird.

Im Fall einer gezielten Verwendung von klassischen Chemotherapien in Kombination mit Antikörper-basierten Immuntherapien, welche im Bereich der Onkologie ein akzeptierter Therapiestandard zur Behandlung verschiedener maligner Erkrankungen, wie z.B. bei Brustkrebs (Trastuzumab), Lymphomen (Rituximab) oder auch bei Darmkrebs (Cetuximab und Panitumumab) sind, wird, wie oben bereits angedeutet, in vivo ebenfalls nicht die erforderliche Wirksamkeit erzielt.

Die synergistische Effektivitätssteigerung der Kombinationstherapie wurde hierbei klinisch und präklinisch beschrieben. Neuere Erkenntnisse basierend auf *in vitro* Experimenten, belegen, dass die Interaktion mit die FcR-positiven Zellen, wie z.B. NK, DC und dergleichen, dem beobachteten Synergismus zu Grunde liegen. Es wurde nachgewiesen, dass es hierbei zum einen durch die Verwendung von Arzneimitteln, wie z.B. Paclitaxel (Miura D. et al., Journal of Clinical Oncology, 2007, Part I. Vol 25, No. 18S (June 20 Supplement), Lenalidomiden und Pomalidomide (Bartlett J.B. et al., Journal of Clinical Oncology, 2007 ASCO Annual Meeting Proceedings Part I. Vol 25, No. 18S (June 20 Supplement)), aber auch Kinaseinhibitoren, wie z.B. Sorafinib (Hipp et al, Journal of Clinical Oncology, 2007 ASCO Annual Meeting Proceedings Part I. Vol 25, No. 18S (June 20 Supplement) einen direkten Einfluss auf die Homeostase der Antigen-präsentierenden Zellen aber auch auf verschiedene T- Zellpopulationen, wie z.B. cytotoxische T-Zellen (CTLs) haben. Für Sorafinib ist hierbei ein negativer Einfluss auf Antigen-präsentierende Zellen und die Entwicklung einer CTL-Antwort nachgewiesen worden, weshalb Kinaseinhibitoren wie Sunitib als geeigneter für eine Kombinationstherapie mit Immuntherapeutika erscheinen, ebenso verschiedene Her2 Kinaseinhibitoren wie z.B. Lapatinip oder auch Canertinib. In den oben zitierten Arbeiteten, wurde der synergistische Effekt auf die FcR-positiven Zellpopulationen zurückgeführt, die die ADCC vermitteln.

Klinisch ist bei den existierenden Kombinationstherapien leider zu verzeichnen, dass eine Mehrheit der behandelten Patienten Resistenzmechanismen gegen die verwendete Chemotherapie und gegen die entsprechende Antikörper basierte Immuntherapie entwickeln. Hierbei spielen unabhängig voneinander verschiedene Mechanismen eine Rolle. Für die oben aufgeführten Immuntherapien sind einige Target-abhängige aber auch Target-unabhängige Resistenzmechanismen beschrieben worden (Herceptin:Ritter C.A. et al.,Clinical Cancer Research 2007, 13(16):4909-4919; Valabrega G. et al., Annals of Oncology, 2007; Nahta R, et al., Natl Clin Pract Oncol 2006, 3:269-280; Esteva FJ et al., J Clin Oncol 2002, 20:1800-1808; Nagata Y et al., Cancer Cell 2004, 6:117-127; Scaltriti M et al., J Natl Cancer Inst 2007, 99:628-638; Rituximab: Van Meerten t et al., Clinical Cancer Research Vol. 12, 4027-4035, July 1, 2006;).

R. A. Montgomery et al. beschreibt (Transplantation, Vol. 70, 887-895, September 27, 2000) ein Therapieverfahren um Abstoßungsreaktionen von Organtransplantaten zu verhindern. Dabei wird eine Plasmapherese (PP) mit intravenösem Gammaglubulin (IVIG) und Cytogam kombiniert. Durch eine Überdosis verabreichter Ig-Antikörper im Blut des Patienten kommt es zu einer Blockade von Fc-Rezeptoren und somit zu einer Immunsuppression. Eine Blockade der Fc-Rezeptoren ist im Sinne der Erfindung jedoch nicht gewünscht, sondern es sollen im Gegensatz dazu gerade die Fc-Rezeptoren von Antikörpern freigehalten werden, damit therapeutische Antikörper an diese Stellen binden und eine ADCC bewirken können. Eine Kombination von PP/IVIG und gegebenenfalls Cytogam soll also ausgeschlossen werden.

H. Borberg offenbart ein Verfahren zur Verringerung von IgG mittels einer anti-IgG Adsorptionssäule (H. Borberg, Transfusion and Apheresis Science, 34, 2006, 51-73) in Kombination mit einer IVIG Verabreichung. Die oben dargestellten Probleme einer Blockierung der Fc-Rezeptoren sind auch hier die Folge.

Das US 6,406,861 B1 offenbart ein Verfahren zur Verringerung von Virusantikörpern, insbesondere von Adenovirusantikörpern, durch extrakorporale Adsorption mit dem Ziel, die Effizienz einer Virusvektor-Therapie zu verbessern. Das US 6,406,861 B1 betrifft also keine Immuntherapie.

EP 0 082 345 A1 betrifft eine therapeutische Vorrichtung zum Entfernen eines gesundheitsschädlichen Mittels aus dem Blut in Form einer Anordnung von Bündeln von Hohlfasern, die parallel angeordnet sind, worin die innere Oberfläche der individuellen Hohlfasern mit einem kovalent gebundenen Protein oder einem anderen Immunadsorbens beschichtet ist, das für die spezifische Entfernung des gesundheitsschädlichen Mittels geeignet ist.

P. Kiewe et al. beschreiben in Clin. Cancer Res. 2006; 12 (10), May 15, 2006 die Wirkungsweise von Ertumaxobab in der klinischen Phase I zur Behandlung von metastasierendem Brustkrebs.

R. P. Taylor et al. beschreiben in Nature Clinical Practice Rheumatology, February 2007, Vol. 3, No. 2, p. 86-95 den Wirkungsmechanismus von Rituximab bei Autoimmunerkrankungen.

J. L. Browning beschreibt in Nature Reviews Drug Discovery July 2006, Bd. 5, Nr. 7, Juli 2006 (2006-07), Seiten 564 bis 576 neue Möglichkeiten von B-Zellen zur Behandlung von Autoimmunerkrankungen.

G. Tyden et al. beschreiben in American Journal of Transplantation 2005; 5; 145 bis 148 die Verwendung von Antigen-spezifischer Immunoadsorption und Rituximab bei ABOinkompatiblen Nierentransplantationen ohne Milzentfernung.

J. Rech et al. beschreiben in Rheumatology, April 2006, Bd. 45, Nr. 4, April 2006, Seiten 490 bis 491 die Kombination von Immunoadsorption und CD20-Antikörpertherapie bei Patienten mit konfusen Erkrankungen.

J. Rech et al. beschreiben in Annals of the Rheumatic Diseases, April 2006, Vol. 65, No. 4, pages 552 to 553 Immunoadsorption und CD20-Antikörperbehandlung bei einem Patienten bei resistenter systemischer Lupus erythematodes und Niereninsuffizienz.

Es bestand daher die Aufgabe der vorliegenden Erfindung insbesondere darin, die Wirksamkeit von Fcγ-R bindenden Agenzien, insbesondere Antikörpern, bei der Behandlung von Krebs zu verbessern, so dass die vorstehend geschilderten Nachteile vermieden werden können.

Es bestand weiterhin die Aufgabe die im Rahmen von Kombinationstherapien aus herkömmlichen Arzneimitteltherapien mit Immuntherapien resultierende Resistenzbildung zu verringern, insbesondere die Effizienz solcher Kombinationstherapien zu steigern.

Gegenstand der vorliegenden Erfindung ist die Verwendung von Fcγ-Rezeptor bindenden Wirkstoffen zur Herstellung eines Medikaments für die Behandlung von Krebs , wobei die Behandlung die Schritte umfasst des:
a) Bereitstellens einer Blutprobe eines Patienten;
b) Unterwerfens der Blutprobe einer Immunapherese, sowie des Verabreichens der so behandelten Blutprobe an den Patienten
c) Verabreichen des Medikaments an den Patienten,
wobei der Fcγ-Rezeptor bindende Wirkstoff ein monoklonaler und/oder rekombinater Anti-körper ist, der sich gegen Krebsassoziierte Antigene richtet.

Als zusätzlicher Schritt d) kann das Verabreichen eines Arzneimittels erfolgen. Dadurch erhält man eine Kombinationstherapie aus einer Arzneimitteltherapie mit einer Immuntherapie.

Als Gegenstand der Erfindung kann somit auch eine Kombinationstherapie bestehend aus Arzneimitteln oder deren Kombinationen, die zusammen mit FcR-bindenden Immunglobulinen, deren Fragmenten oder auch Fusionsproteinen eine ADCC vermittelte Effektivität aufzeigt, und die deutliche Synergien in der antitumoralen Wirkung aufweist, gesehen werden.

FcR- oder Fcγ-bindende Wirkstoffe im Sinne dieser Erfindung sind solche Wirkstoffe mit einer Bindungsaffinität mit einem Kd-Wert von kleiner als 1 mM.

Vorzugsweise soll die Kombinationstherapie mit Immunglobulinen, deren Fragmenten oder auch Fusionsproteinen, die gezielt CD16 oder CD64 binden, erfolgen.

Weiterhin bevorzugt ist, dass die Immunglobuline, deren Fragmente oder Fusionsproteine neben der ADCC eine T-zellvermittelte Immunantwort induzieren.

Noch weiter bevorzugt ist, dass die Immunglobuline, deren Fragmente oder Fusionsproteine bispezifische Antikörper mit einem CD3 Bindungsarm beinhalten.

Ebenfalls bevorzugt ist eine Kombinationstherapie mit Immunglobulinen, deren Fragmenten oder auch Fusionsproteinen, bei der das Auftreten von Resistenzen durch die vorher genannten antitumoralen Zellantworten vermieden wird. Bevorzugte Arzneimittel für das erfindungsgemäße Verfahren oder die erfindungsgemäße Verwendung sind Cytostatika und Chemotherapeutika, insbesondere Paclitaxel, Lenalidomide, Pomalidomide, Epirubicin, 5FU und dessen Derivate, und Kinaseinhibitoren wie Sunitinib, Lapatinib, Canertinib. Weiterhin können auch Kombinationen aus verschiedenen Arzneimitteln wie CHOP, Cyclophosphamide, Doxorubicin, Vincristin Prednisolon (Steroid), Lenalidomiden/Dexamethason, Pomalidomid/Dexamethason und Paclitaxel/Carboplatin verwendet werden.

Bei den erfindungsgemäßen Verfahren oder Verwendungen können Immunglobuline mit humanen, chimären, murinen, und hybrid Immunglobuline, deren Fragmente oder auch Fusionsproteine, die CD16/CD64 Bindungseigenschaften besitzen, eingesetzt werden. Weiterhin bevorzugt sind Immunglobuline, welche gegen tumorassoziierte Antigene gerichtet sind, sowie gegen Antigene, die mit Lymphomen oder Leukämie assoziiert sind. Diese sind Her2/neu, EGFR, Epcam, VEGF, VEGFR,MUC-1,CA 125,CEA, MAGE, CD20, CD19, CD40, CD33, Kohlenstoffanhydrase IX, A3, Antigen spezifisch für A33 Antikörper, BrE3-Antigen, CD1, CDIa, CD4, CD5, CD8, CD14, CD15, CD16, CD21, CD22, CD23, CD25, CD30, CD37, CD38, CD40, CD40L CD45, CD 46, CD52, CD54, CD74, CD79a, CD80, CD126, CD138, CD154, B7, Ia, Ii, HM1.24, HLA-DR, NCA95, NCA90, HCG und Untereinheiten, CEA (CEACAM-5), CEACAM-6, CSAp, EGFR, EGP-I, EGP-2, Ba 733, Hypoxia induzierender Faktor (HIF), KC4-antigen, KS-I-antigen, KS1-4, Le-Y, Macrophagen inhibierender Faktor (MIF), MUC2, MUC3, MUC4, P1GF, ED-B Fibronectin, NCA 66a-d,PAM-4-Antigen, PSA, PSMA, RS5, SlOO, TAG-72, T101, TAG TRAIL-R1, TRAIL-R2, p53, Tenascin, IL-6, IL-8, Insulin Wachstumsfaktor-1 (IGF-I), Tn Antigen.

Ebenfalls Gegenstand der Erfindung ist eine Kombinationstherapie aus Immuntherapien und immunologisch aktiven Verfahren, die direkt FcR-positive Zellen beeinflussen, indem das Verfahren die Immunglobulinkonzentration im Blut senkt. Besonders bevorzugt ist, wenn das Verfahren, das die Immunglobulinkonzentration senkt, ein extrakorporales Verfahren ist. Weiterhin bevorzugt ist, wenn das Verfahren ein Adsorptionsverfahren ist, das die natürlich vorkommenden Immunglobuline und Immunglobulinkomplexe bindet. Das Verfahren soll darüber hinaus einen Synergismus zwischen den verwendeten Arzneimitteln, deren Kombinationen und den Immuntherapien aufweisen.

Im jeweiligen Schritt b) des oben genannten Verfahrens oder des Verwendung werden durch die Immunapherese der Blutprobe körpereigene Antikörper bzw. Fcγ-R bindende Agenzien entzogen.

Die Entfernung von humanen Anti-Spezies-Antikörpern und IgG-Antikörpern aus dem Patienten vor der Verabreichung des therapeutischen Antikörpers und im Falle einer Kombinationstherapie auch vor der Verabreichnung des Arzneistoffs, führt überraschenderweise dazu, dass die Wirksamkeit der Antikörpertherapie sowie auch der Kombinationstherapie um ein Vielfaches verbessert werden kann, und die Wirksamkeit *in vivo* nunmehr nahezu der erwarteten Wirksamkeit entspricht, wie sie *in vitro* bestimmt wurde. Die Dosierung therapeutischer Antikörper kann mindestens um das 5-fache, bevorzugt um das 10-fache in ganz besonders bevorzugten Ausführungsformen um das 20-fache vermindert werden, verglichen mit der Dosierung therapeutischer Antikörper bei herkömmlichen Verfahren.

Die Kombination aus Arzneimitteln mit Immuntherapien, die neben dem tumorassoziierten Antigen, gezielt FcR-positive Zellen binden, überwinden dadurch eine mögliche Resistenzentwicklungen, die durch die Antigene (Her2/EGFR/CD20), sekundäre intrazelluläre Signaltransduktionskaskaden (Apoptose oder PTEN-Verlust), oder andere Kinaseaktivitäten (HER3/PI3K/Akt) vermittelt sein könnten. Diese Immuntherapien rekrutieren gezielt Antigen-präsentierende Zellen, wodurch wiederum über co-stimulatorische (CD28/CD40) Signale T-Zellen aktiviert werden.

Ideale Kandidaten für eine Kombinationstherapie stellen humanisierte komplette und IgG-ähnliche bispezifische Antikörper (Asano et al., 2007, JBC) und trifunktionale bispezifische Antikörper, vorzugsweise Maus/Ratte IgG2a/IgG2b Chimäre, dar. Bei diesen Maus/Ratte IgG2a/IgG2b Chimären werden durch den zweiten CD-3-Bindungsarm sogar noch gezielt T-Zellen an die FcR I und III-positiven Zellen gebunden. Beide Rezeptoren zählen zu den ADCC vermittelnden Rezeptoren.

Neben den trifunktionalen Antikörpern, existieren bereits verschiedene andere Immuntherapien, die gezielt CD16 oder auch CD64 positive Zellen rekrutieren (Her2/CD16; CD30/CD16; CD19/CD64; CD15/CD64; Her2/CD64 u.s.w.) oder durch verbesserte FcR Bindungseigenschaften eine Verstärkung der ADCC ermöglichen. Auch solche Immuntherapien stellen geeignete Kandidaten für eine Kombinationstherapie dar, bei der potentielle Resistenzmechanismen umgangen und eine synergistische Therapie konzipiert werden kann.

Die bereits beschriebenen synergistischen Effekte einer Kombinationstherapie bestehend aus den oben genannten Arzneimitteln und der Immuntherapie, lässt sich mit dem oben beschriebenen Verfahren der Immunadsorption noch weiter verstärken. Die gezielte Abreicherung der natürliche vorkommenden Immunglobuline durch eine extrakorporale Immunadsorption soll hierbei vor der Kombinationstherapie erfolgen. Dabei würde die ADCC noch weiter verstärkt werden, da die Konzentration der Immunglobuline herabgesetzt und damit mehr FcR auf den Zelloberflächen in ungebundenen Zustand zur Verfügung stehen. Der synergistische Einfluss des Immunadsorptionsverfahren kann hierbei für die Verwendung mit Arzneimitteln als Mono- und/oder Kombinationstherapien genutzt werden. Als Beispiel hierfür sind Sunitinib oder auch Paclitaxel/Carboplatin bzw. Lenalidomid/Dexamethason zu nennen.

Die Arzneimittel/Immuntherapie-Kombinationen lassen sich mit diesem Verfahren weiter verstärken. Einen besonders starken Synergismus ergibt sich jedoch aus Zusammenspiel mit der Kombinationstherapie bestehend aus Arzneimitteln mit FcR gerichteten Immuntherapien.

Eine Kombinationstherapie mit humanisierten und IgG-ähnlichen bispezifischen Antikörpern und/oder trifunktionalen Antikörpern und einer vorgeschalteten Immunadsorption bewirkt nicht nur eine stärkere Synergie bezüglich der ADCC und T-Zellvermittelten Cytotoxizität, sondern ermöglicht auch die Langzeitbehandlung der Kombinationstherapie mit den murinen Antikörpern. Die vorgeschaltete Immunadsorption neutralisiert auch die potentiell neutralisierenden Immunglobuline (HAMA, ADA), die sich gegen den therapeutischen Antikörper richten.

Durch die Entfernung der körpereigenen Antikörper fällt die Immunreaktion gegen die therapeutischen Antikörper wesentlich milder aus und die Wirksamkeit der therapeutischen Antikörper wird dadurch *in vivo* überraschenderweise gesteigert.

Mit Fcγ-Rezeptoren wechselwirkende Moleküle sind z. B. Immunoglobuline oder auch das pro-inflammatorische C-reaktive Protein (Das, T., FEBS Lett., 2004), die die zur Verfügung stehenden Fcy-Rezeptoren besetzen.

Ein erfindungsgemäßes Beispiel ist die Antikörper-vermittelte zelluläre Zytotoxizität (ADCC) die für die Wirkung biologischer Arzneimittel, wie z.B. monoklonale Antikörper, aber auch von Fusionsproteinen verantwortlich. Die Wirksamkeit der ADCC steht in direktem Zusammenhang mit der Wechselwirkung der konstanten Region des Antikörpers aber auch mit den Bindungseigenschaften des entsprechenden Proteins mit den Fcy-Rezeptoren. Für die ADCC scheint hierbei vor allem die Bindung an die aktivierenden FcyI- und III-Rezeptoren relevant zu sein. Die Bindung eines Arzneimittels vorwiegend an FcγII-Rezeptoren unterdrückt hingegen die Immunantwort. Beispielsweise gibt es bi-spezifische Antikörper, die gegen ein Target-Protein wie z.B. HER2 aber auch gleichzeitig gegen den Fcγ-Rezeptor I oder den Fcγ-Rezeptor III gerichtet sind.

Weitere bevorzugte Wirkstoffe, die Fcy-Rezeptoren binden, sind typischerweise monoklonale Antikörper tierischen und humanen Ursprungs, rekombinante Antikörper, chimäre, primatisierte, humanisierte und humane monoklonale Antikörper, Antikörperdomänen und Fragmente davon, bi-, tri- und multispezifische Antikörper und Konstrukte von Antikörperfragmenten.

Beispielsweise verdeutlicht die Entwicklung von Impfstrategien unter Ausnutzung des gezielten Ansteuerns von Fcy-Rezeptor-positiven Dendritischen Zellen, z.B. durch die Kopplung von DNA Impfstoffen an IgG Strukturen (Zhaoyang You et al., 2001, Cancer Research) die Bandbreite möglicher Anwendungen, die sich durch Fc-Rezeptoren und daran bindende Agenzien eröffnen.

Durch den Entzug der mit Fcγ-Rezeptoren wechselwirkenden Moleküle im Blut gibt es für einen anschließend verabreichten Fcγ-R bindenden Wirkstoff einen geringeren Wettbewerb um die Bindungsstellen, so dass die Fcγ-R bindenden Wirkstoffe nunmehr bevorzugt binden können.

Nach der Behandlung mit therapeutischen Antikörpern bzw. mit Fcy-Rezeptoren bindenden Wirkstoffen, bzw. nach der Kombinationstherapie, können dem Patienten in bevorzugten Ausführungsformen des erfindungsgemäßen Verfahrens die körpereigenen Antikörper beispielsweise im Anschluss an die Verabreichung der Fc-R bindenden Agenzien ebenfalls rückgeführt werden.

Das Verfahren kann sowohl kontinuierlich, d.h. extrakorporal in einem Kreislauf, wie auch diskontinuierlich durchgeführt werden, wonach dem Patienten jeweils Blut abgezapft, dieses dem erfindungsgemäßen Verfahren unterzogen und anschließend dem Patienten rückgeführt wird.

Die zeitweise Entfernung von humanen Anti-Spezies Antikörpern, wie z.B. HAMA und HARA sowie von IgG-Antikörpern im Allgemeinen ist für den Patienten sicher und erhöht nicht das Risiko für Infektionen.

Schritt b) des erfindungsgemäßen Verfahrens wird in bevorzugten Weiterbildungen mehrfach durchgeführt. Dabei können bis zu 80%, bevorzugt 90%, noch mehr bevorzugt bis zu 95% der entsprechenden Antikörper bzw. Fcγ-Rezeptor bindenden Wirkstoffe aus dem Blut des Patienten entfernt werden.

Erfindungsgemäß kann die Blutprobe menschliches Blut oder Blutplasma sein. Das Plasma kann dabei in einer vorgeschalteten Stufe z. B. durch Plasmafiltration oder Zellseparation durch Zentrifugieren des Blutes erhalten werden.

So kann beispielsweise das Blut des Patienten bzw. das Plasma auch in einem extrakorporalen Schritt über einen Adsorber geleitet werden, der Fcγ bindende Agenzien, insbesondere Antikörper, bindet. Das Blut oder Plasma kann dem Patienten anschließend rückgeführt werden. Weiterhin kann durch die Verwendung spezifischer Liganden für körpereigene Antikörper mit Fcγ-Rezeptor-bindenden Regionen eine Säule hergestellt werden, die den spezifischen Liganden daran gekoppelt aufweist, für die Behandlung eines Patienten mit Fcy-Rezeptor-bindenden Wirkstoffen, wobei die Behandlung die Schritte umfasst:
a) des Bereitstellens einer Blutprobe eines Patienten;
b) des Unterwerfens der Blutprobe einer Immunapherese;
c) des Verabreichens eines Fcy-Rezeptor-bindenden Wirkstoffs an den Patienten.

Sowie vor oder nach Schritt c) optional das Verabreichen der so behandelten Blutprobe an einen Patienten.

Unter dem Begriff "spezifische Liganden" werden solche Liganden verstanden, die selektiv Antikörper aus dem Blut entfernen, jedoch nicht andere Bestandteile des Blutes.

Als spezifischer Ligand kann beispielsweise Protein A verwendet werden, bzw. Moleküle, die Fcγ-Rezeptoren in ihrer Wirkung äquivalent sind, Fragmente davon, künstliche Peptide, Proteine, usw. Weitere Beispiele sind nachstehend angegeben.

Weiterhin kann durch die Verwendung spezifischer Liganden für körpereigene Anti-körper eine Säule hergestellt werden, die den Liganden daran gekoppelt aufweist, für die Behandlung eines Patienten mit therapeutischen Antikörpern, wobei die Behandlung die Schritte umfasst:
a) des Bereitstellens einer Blutprobe eines Patienten;
b) des Unterwerfens der Blutprobe einer Immunapherese;
c) des Verabreichens eines Fcγ-Rezeptor-bindenden Wirkstoffs an den Patienten.

Sowie vor oder nach Schritt c) optional das Verabreichen der so behandelten Blutprobe an einen Patienten.

Die Matrix der verwendeten Säule für die Immunapherese besteht dabei aus Sepharose oder Acrylverbindungen, wie sie z. B. in dem EP 222 146 B1 beschrieben sind.

Vor Aufbringung des Liganden wird die Matrix bevorzugt mit CN-Br oder ähnlich wirkenden Verbindungen aktiviert.

Bevorzugt werden als Liganden Protein A, Protein G, Peptide, oder AntiAntikörper verwendet. Weiterhin kommen als Liganden FcR-Rezeptoren, Fragmente davon, oder äquivalente natürliche oder synthetische Moleküle mit gleichwirkenden Bindungseigenschaften in Frage.

Weiterhin kann die extrakorporale Entfernung von humanen Anti-Spezies Antikörpern und IgG-Antikörpern durch ein Kombinationssystem erreicht werden, das aus einer Vorrichtung für die Blutplasmaerzeugung und einer zweiten Vorrichtung, in der das Plasma über eine Adsorber-Kolonne geführt wird, besteht. Diese Adsorber-Säule arbeitet im Prinzip wie eine Chromatographie-Säule.

Die Adsorber-Säule besteht typischerweise aus einem biokompatiblem Plastikgehäuse und enthält 20 bis 1500 ml einer inerten Matrix, auf der spezifische Liganden mit einer Affinität beispielsweise gegenüber humanen Anti-Spezies-Antikörpern wie HAMA, HARA und menschlichem IgG1 immobilisiert sind.

Wenn das Plasma über die Adsorber-Kolonne geführt wird, werden die humanen Anti-Spezies-Antikörper und IgGl-Antikörper aus dem Patientenblut durch diese Liganden gebunden und daher aus dem Plasma eliminiert.

Derartige Systeme bzw. Materialien sind beispielsweise aus der EP 0 082 345 bekannt.

Die Adsorber-Säulen sind regenerierbar und können wiederholt verwendet werden.

In einem typischen Zweisäulensystem ist nur eine Säule in Gebrauch, wohingegen die zweite Säule automatisch regeneriert wird. Auf diese Weise kann die Geschwindigkeit für die Verminderung der abzureichernden Spezies während einer einzigen Behandlungssitzung um bis zu 60 % gesteigert werden.

Alternativ dazu können auch größere Säulen mit mehr selektiven Liganden und einer höheren Adsorptionskapazität als Einfach-Kolonnen verwendet werden.

Weiterhin kann durch die Verwendung spezifischer Liganden für Krebs-assoziierte Antigene (Krebsmarker), wobei der Krebsmarker im Blut löslich ist, eine Säule hergestellt werden, die den spezifischen Liganden daran gekoppelt aufweist, für die Behandlung eines Patienten mit tumorspezifischen Antikörpern, wobei die Behandlung die Schritte umfasst:
a) Bereitstellen einer Blutprobe eines Patienten;
b) Unterwerfen der Blutprobe einer Apherese unter Entfernung der Krebs-assoziierten Antigene;
c) Verabreichung eines therapeutisch wirksamen Antikörpers an den Patienten.

Sowie vor oder nach Schritt c) optional das Verabreichen der so behandelten Blutprobe an einen Patienten.

Antikörper-basierte Therapien, die sich gegen lösliche und Zellmembran gebundene Krebs-assoziierte Antigene richten, befinden sich derzeit in der klinischen Evaluierung. Zu solchen Krebs-assoziierten Antigenen gehören unter anderem CH125, PSA, MUC1, MAGE-1, HER2, CEA, AFP, EpCAM. Der verwendete spezifische Ligand besitzt eine hohe spezifische Bindungsaffinität zu dem Krebs-assoziierten Antigen. Insbesondere spezifische Antikörper gegen Krebs-assoziierte Antigene können als Liganden verwendet werden.

Die spezifische Adsorption der löslichen Krebs-assoziierten Antigene führt dazu, dass die therapeutischen Antikörper oder auch Peptide an der Komplexierung mit frei löslichen Antigenen gehindert werden. Auf diese Weise wird die Wirkstoffkonzentration an der Zielstruktur, dem Antigen auf der Zelle, erhöht und somit die Wirksamkeit erhöht. Außerdem kann damit die Antikörper-vermittelte, zelluläre Zytotoxizität (ADCC) als primäre, gegen den Antigen-positiven Tumor gerichtete, und damit äußerst effiziente Immunantwort, ermöglicht werden. Weiter führt die spezifische Adsorption, beispielsweise von PSA, zu einer Verstärkung der Wirksamkeit löslicher T-Zell-Rezeptoren, die spezifisch an PSA binden.

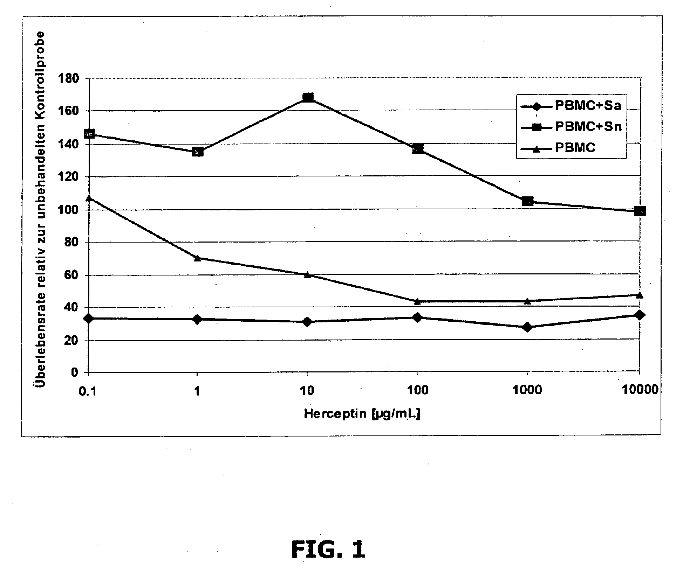

Die Erfindung wird weiter anhand eines nicht einschränkenden Beispiels unter Bezugnahme auf Figur 1 weiter erläutert.

### Beispiel 1:

### Aufhebung der Seruminhibition von Herceptin-vermittelter ADCC durch IgG-Adsorption:

Experimenteller Aufbau:
1. Aussaat von Tumorzellen (Her-2/neu positive SK-OV-3 Ovarialzellen) über Nacht;
2. Isolation von PBMC (periphere mononukleare Blutzellen) aus dem buffy coat (Leukozytenfilm) ;
3. Co-Kultivierung von PBMC und Tumorzellen mit Herceptin unter Anwesenheit von IgG-depletierten (Sa) und nativem Serum (Sn);
4. Die Herceptin-Konzentrationen betragen 0,0001, 0,001, 0,01, 0,1, 1 und 10 µg/ml;
5. Die Inkubationszeit betrug 20 Stunden;
6. Die Messung der Zytotoxizität erfolgte mittels XTT;

Figur 1 zeigt, dass die Zytotoxizität der PBMC durch Zugabe von normalem, humanem Serum (Kurve PBMC + Sn) aufgehoben wird. Nach Zugabe von IgG-depletiertem Plasma (Kurve PBMC + Sa) bleibt die Zytotoxizität der PBMC erhalten. Im unteren Bereich der Herceptinkonzentration wird sie noch verstärkt.

## Patentansprüche

1. Verwendung von einem Fcγ-Rezeptor bindenden Wirkstoffen zur Herstellung eines Medikaments für die Behandlung von Krebs, wobei die Behandlung die Schritte umfasst des:
a) Bereitstellens einer Blutprobe eines Patienten;
b) Unterwerfens der Blutprobe einer Immunapherese sowie des Verabreichens der so behandelten Blutprobe an den Patienten;
c) Verabreichens des Medikaments an den Patienten,
und wobei der Fcγ-Rezeptor bindende Wirkstoff ein monoklonaler und/oder rekombinanter Antikörper ist, der sich gegen Krebsassoziierte Antigene richtet.

2. Verwendung nach Anspruch 1, **dadurch gekennzeichnet, dass** als zusätzlicher Schritt d) das Verabreichen eines Arzneimittels erfolgt.

3. Verwendung nach Anspruch 2, **dadurch gekennzeichnet, dass** das Arzneimittel ein Chemotherapeutikum oder Cytostatikum ist.

4. Verwendung nach Anspruch 2 oder 3, **dadurch gekennzeichnet, dass** das Arzneimittel ausgewählt ist aus einer Gruppe umfassend Paclitaxel, Lenalidomid, Pomalidomid, Epirubicin, 5FU und dessen Derivaten, und Kinaseinhibitoren wie Sunitinib, Lapatinib, Canertinib, oder Kombinationen aus verschiedenen Arzneimitteln, ausgewählt aus der Gruppe umfassend CHOP, Cyclophosphamid, Doxorubicin, Vincristin, Prednisolon (Steroid), Lenalidomiden/Dexamethason, Pomalidomid/Dexamethason und Paclitaxel/Carboplatin.

5. Verwendung nach Anspruch 1, **dadurch gekennzeichnet, dass** die in Schritt b) der Blutprobe entzogenen Anti-körper dem Patienten im Anschluss an Schritt c) wieder verabreicht werden.

6. Verwendung nach einem der Ansprüche 1 bis 5, **dadurch gekennzeichnet, dass** die Blutprobe menschliches Blut oder Blutplasma ist.

7. Fcγ-Rezeptor bindender Wirkstoff zur Verwendung in der Behandlung von Krebs, wobei die Behandlung die Schritte umfasst des:
a) Bereitstellens einer Blutprobe eines Patienten;
b) Unterwerfens der Blutprobe einer Immunapherese sowie des Verabreichens der so behandelten Blutprobe an den Patienten;
c) Verabreichens des Medikaments an den Patienten,
und wobei der Fcγ-Rezeptor bindende Wirkstoff ein monoklonaler und/oder rekombinanter Antikörper ist , der sich gegen Krebsassoziierte Antigene richtet

## Claims

1. Use of an Fcγ receptor-binding active ingredient to produce a medicament for the treatment of cancer, wherein the treatment comprises the steps of:
a) preparing a blood sample of a patient;
b) subjecting the blood sample to an immunoapheresis as well as administering the thus-treated blood sample to the patient;
c) administering the medicament to the patient,
and wherein the Fcγ receptor-binding active ingredient is a monoclonal and/or recombinant antibody which is directed against cancer-associated antigens.

2. Use according to claim 1, **characterized in that** the administration of a medicinal product takes place as additional step d).

3. Use according to claim 2, **characterized in that** the medicinal product is a chemotherapeutic agent or cytostatic agent.

4. Use according to claim 2 or 3, **characterized in that** the medicinal product is selected from a group comprising paclitaxel, lenalidomide, pomalidomide, epirubicin, 5FU and its derivatives, and kinase inhibitors such as sunitinib, lapatinib, canertinib, or combinations of various medicinal products, selected from the group comprising CHOP, cyclophosphamide, doxorubicin, vincristine, prednisolone (steroid), lenalidomide/dexamethasone, pomalidomide/dexamethasone and paclitaxel/ carboplatin.

5. Use according to claim 1, **characterized in that** the antibodies extracted from the blood sample in step b) are readministered to the patient after step c).

6. Use according to one of claims 1 to 5, **characterized in that** the blood sample is human blood or blood plasma.

7. Fcγ receptor-binding active ingredient for use in the treatment of cancer, wherein the treatment comprises the steps of:
a) preparing a blood sample of a patient;
b) subjecting the blood sample to an immunoapheresis as well as administering the thus-treated blood sample to the patient;
c) administering the medicament to the patient,
and wherein the Fcγ receptor-binding active ingredient is a monoclonal and/or recombinant antibody which is directed against cancer-associated antigens.

## Revendications

1. Utilisation d'un agent de liaison au récepteur Fcγ pour la fabrication d'un médicament pour le traitement du cancer, dans laquelle le traitement comprend les étapes de :
a) mise à disposition d'un échantillon sanguin d'un patient ;
b) soumission de l'échantillon sanguin à une immunophorèse ainsi qu'administration de l'échantillon sanguin ainsi traité aux patients ;
c) administration du médicament aux patients,
et dans laquelle l'agent de liaison au récepteur Fcγ est un anticorps monoclonal et/ou recombiné qui se dirige contre des antigènes associés au cancer.

2. Utilisation selon la revendication 1, **caractérisée en ce que** l'administration d'un produit pharmaceutique est effectuée en tant qu'étape supplémentaire d).

3. Utilisation selon la revendication 2, **caractérisée en ce que** le produit pharmaceutique est un agent de chimiothérapie ou cytostatique.

4. Utilisation selon la revendication 2 ou 3, **caractérisée en ce que** le produit pharmaceutique est sélectionné parmi un groupe comprenant paclitaxel, lénalidomide, pomalidomide, épirubicine, 5FU et ses dérivés, et des inhibiteurs de kinase comme le sunitinib, lapatinib, canertinib, ou des combinaisons de différents produits pharmaceutiques sélectionnés parmi le groupe comprenant CHOP, cyclophosphamide, doxorubicine, vincristine, prednisolone (stéroïde), lénalidomide/dexaméthasone, pomalidomide/dexaméthasone et paclitaxel/carboplatine.

5. Utilisation selon la revendication 1, **caractérisée en ce que** les anticorps prélevés de l'échantillon sanguin à l'étape b) sont de nouveau administrés au patient suite à l'étape c).

6. Utilisation selon l'une quelconque des revendications 1 à 5, **caractérisée en ce que** l'échantillon sanguin est du sang humain ou du plasma sanguin.

7. Agent de liaison au récepteur Fcγ pour l'utilisation dans le traitement du cancer, dans lequel le traitement comprend les étapes de :
a) mise à disposition d'un échantillon sanguin d'un patient ;
b) soumission de l'échantillon sanguin à une immunophorèse ainsi qu'administration de l'échantillon sanguin ainsi traité aux patients ;
c) administration du médicament aux patients,
et dans lequel l'agent de liaison au récepteur Fcγ est un anticorps monoclonal et/ou recombiné qui se dirige contre des antigènes associés au cancer.
